# EUROPEAN PATENT SPECIFICATION

(11) **EP 0 506 860 B1**
(45) Date of publication and mention of the grant of the patent: **13.05.1998**
(21) Application number: 91902617.9
(22) Date of filing: 17.12.1990
(51) Int. Cl.: B32B 27/30, B32B 15/08, B32B 27/10, B32B 27/36

(54) **NICOTINE PACKAGING MATERIALS**
NIKOTINVERPACKUNGSMATERIALIEN
MATERIAUX POUR EMBALLER DE LA NICOTINE

(30) Priority: 21.12.1989 US 454257
(43) Date of publication of application: 07.10.1992
(73) Proprietor: ALZA CORPORATION, Palo Alto California 94303-0802 (US)
(72) Inventor: HUNT, James, A., Fremont, CA 94536 (US); OSBORNE, James, L., Mountain View, CA 94043 (US); DUNN, John, Thomas, Redwood City, CA 94061 (US); NELSON, Melinda, K., Sunnyvale, CA 94087 (US); ROTH, Nathan, San Francisco, CA 94133 (US)
(74) Representative: Evans, David Charles
(86) International application number: PCT/US90/07543
(87) International publication number: WO 91/09731

(56) References cited:
- EP-A- 0 273 004
- EP-B- 0 075 486
- Derwent Accession No. 80-79 937 (WPI) & JP,A, 55/124 654 (NITTO ELECTRIC IND. KK) 26 September 1980 (26.09.80).

## Description

The invention herein pertains to materials and methods for the manufacture of nicotine storage pouches.

Nicotine containing products are known to the art for numerous purposes including swine and food additives, U.S. Patent 3,252,802; dosage forms for the treatment of mental conditions, U.S. Patent 3,870,794 and 4,680,172; pain, U.S. Patent 4,665,069; hypertension, U.S. Patent 4,748,181; and nicotine addiction, U.S. Patents 3,877,468, 3,845,217, 4,597,961, 4,715,387, 4,758,434, 4,839,174 and 4,943,435 for example.

Nicotine base, however, is a reactive species which degrades in the presence of oxygen or light and is highly hygroscopic. Nicotine base also causes the solvation of adhesives typically used in the manufacturer of pouch stock which prevents their effective use when exposed to nicotine.

The problem of providing containment for products containing nicotine base is described in patents 4,839,174 and 4,943,435 and has heretofore found no simple solution. For example, transdermal systems for the delivery of nicotine are disclosed in U.S. patents 4,781,924 and 4,837,027 and Australian Patent Application Au-A-81454/87. All of the above noted U.S. patents are incorporated herein by reference. In those systems nicotine precursors instead of nicotine base are in the devices prior to use and the precursors are converted to the highly reactive nicotine immediately prior to or after application to the skin thereby avoiding the problems associated with the long term storage of nicotine. A commercially available nicotine releasing chewing gum is available as Nicorette® (Lakeside Pharmaceuticals). This dosage form is packaged in a foil-plastic blister pouch, however, prior to use, the nicotine is chemically bound within a polymeric material in the form of nicotine polacrilex.

DE-OS-3629304 discloses a nicotine patch which is packaged prior to use by sealing in a bag made from a paper-aluminium-Barex composite.

There is a need therefore, to provide a container in the form of pouch or blister pack, for example, which can hold one or more nicotine-containing devices in a stable manner for extended periods of time. To function properly such a container should be inert to nicotine; that is, it would not cause degradation of nicotine base, or be degraded by nicotine. The container should also act as a barrier to nicotine and to agents which are detrimental to the stability of nicotine base such as air, moisture and light.

Air (oxygen) and light both act to degrade nicotine base. Since nicotine is strongly hygroscopic, nicotine tends to absorb water in liquid or vapor form which would cause a change in the concentration or thermodynamic activity of the nicotine in the product and thereby effect its performance.

Materials which were known, or could be reasonably expected, to provide a barrier to nicotine and provide protection from nicotine degradation agents include metal foils such as aluminum foil, polyesters such as polyethylene terephthalate (PET), and fluorinated polymers such as poly(tetrafluorethylene) (PTFE). However, these materials require the use of an adhesive to bond opposing surfaces and we have found that the adhesive itself can provide a path for migration of nicotine out of the package and/or migration of degradation agents into the package. We have also found that many self-sealable pouching materials of the prior art such as polyvinyl fluoride (PVF), polyvinylidene fluoride (PVDF), Chlorotrifluoroethylene (CTFE), low density polyethylene (LDPE), and high density polyethylene (HDPE) do not provide an effective barrier for nicotine or its degradation agent.

As a result, prior to our invention, a nicotine containing pouch or package capable of enclosing nicotine in a stable manner, over a long period of time, was not available.

According to one aspect of the present invention therefore there is provided a laminate pouch for the packaging of nicotine or a nicotine device, which pouch is made from a composite of nitrile rubber modified acrylonitrile-methyl acrylate copolymer and an opaque material impermeable to nicotine degrading agents, characterised in that said laminate comprises;
(i) first and second layers of said copolymer, each layer having a first and a second surface, and wherein portions of the second surface of the first layer juxtaposed the outer edges thereof are disposed in juxtaposition with corresponding portions of the second surface of the second layer in self-sealing contact, thereby to define self-sealed portions of an enclosure for said nicotine or nicotine device, which enclosure lies between said first and second layers, and
(ii) top and bottom layers of said opaque material, which top and bottom layers are juxtaposed in adhesive contact with the respective first surfaces of said copolymer first and second layers,
the arrangement being such that said self-sealed portions are unprotected by said opaque material.

It is therefore an object of this invention to provide a container for nicotine base or for products containing nicotine base.

It is another object of the invention to provide a pouching material which acts as a barrier to nicotine and nicotine-degradation agents such as air (oxygen), water in liquid or vapor form, and light.

It is yet another object of the invention to provide a pouch which is suitable for long-term containment of nicotine-containing transdermal delivery devices.

Following is a description by way of example only with reference to the accompanying drawings of methods of carrying the present invention into effect.

In the drawings:
Figure 1 shows a top view of a pouch of this invention.
Figure 2 shows a cross-sectional representation of a laminate useful to form a pouch of Figure 1.
Figure 3 shows a cross section of the pouch of Figure 1 taken through line A-A. The pouch has been formed of the laminate of Figure 2.
Figures 4 and 8 show representations of the laminates used to form the pouches of Figures 5 and 9 respectively.
Figure 9 shows a view of an alternate embodiment of a pouch such as that shown in Figure 1.
Figure 12 shows comparative nicotine transmission data for various barrier materials.

The invention herein pertains to methods and materials for producing a package for enclosing a nicotine containing product in a stable manner over an extended period of time of at least one year and preferably at least two years.

Laminates comprising a nicotine barrier layer and a nicotine degradation agent barrier layer are disclosed. The nicotine barrier layer of this invention comprises a nitrile rubber modified acrylonitrile-methyl acrylate copolymer (hereafter "AN-MA/B"). The preferred material comprises the material produced by the graft copolymerization of about 73-77 parts by weight (pbw) acrylonitrile and about 23-27 pbw of methyl acrylate in the presence of about 8-10 pbw of butadiene-acrylonitrile copolymer containing approximately 70 wt % of polymer units derived from butadiene (hereafter "73-77 AN-MA/B"). An especially preferred nicotine barrier material comprises the graft copolymer of 75 pbw acrylonitrile, and 25 pbw methyl acrylate in the presence of 10 pbw of the butadiene-acrylonitrile copolymer (hereafter "75AN-MA/B"). A preferred nicotine degradation agent barrier layer is a metal film, preferably aluminum. A preferred embodiment of the pouch stock of this invention comprises a laminate of a layer of 75AN-MA/B adhered to a layer of aluminum foil, which may optionally be provided with an external protective layer formed from paper, polymeric materials or the like.

In yet another aspect, the invention includes a method for the production of the pouches of this invention which comprises: (a) providing a laminate including a self-sealing nicotine barrier layer and a nicotine degradation agent barrier layer; (b) depositing a source of nicotine on said laminate; (c) enclosing said source within said laminate; and (d) sealing the self-sealing nicotine barrier layer.

Nicotine (beta-pyridyl-alpha-N-methylpyrrolidine) is a colorless liquid alkaloid derived from tobacco or by synthetic manufacture. It is a strongly alkaline, hygroscopic liquid which degrades upon exposure to air (oxygen) or light, the principal degradation products being cotinine and the cis and trans N-oxides of nicotine.

As used herein, the term "pouch" refers to a pouch package, blister pack or other container for nicotine which is sealed on at least one side. The sealed pouches of this invention can comprise, for example, two sheets of the above described laminates which have been joined along all edges; a single sheet of the laminate which has been folded and sealed along all edges or along all non-fold edges; a bag or pocket which is sealed along one or more edges; and the like.

"Self-sealing" refers to the ability of a material to form a stable bond between one face of the material and another face of the same material. Self-sealing is accomplished, for example, by heat (thermal bar) sealing, impulse, radio frequency, ultrasonic sealing, and the like.

As used herein, a "nicotine barrier" is a material that permits, at ambient conditions (30°C or below), less than 1% by weight of the nicotine content of the packaged product to migrate over a period of six months and more preferably less than 0.5% by weight and most preferably substantially no nicotine migration. The nicotine barrier material is also inert with respect to nicotine, i.e., nicotine does not act as a solvent or plasticizer for the barrier material, does not cause stress cracking, crazing or otherwise adversely affect the physical characteristics of the barrier material and the barrier material does not cause the nicotine to degrade.

As used herein, "nicotine degradation agents" refer to air (particularly oxygen), water in liquid or vapor form, and light. A "nicotine degradation agent barrier" is a barrier which is opaque and substantially impermeable to nicotine degradation agents.

"Nicotine containment" refers to the long-term, at least six months at ambient conditions, storage of nicotine base especially as embodied in a nicotine device, without significant migration of the nicotine out of the storage container, and without significant degradation of the nicotine by oxygen, water, or light.

As used herein, significant degradation of the nicotine is considered to have occurred if the nicotine component of the device contains 3 wt% or more of total impurities, including water and nicotine degradation products such as cotinine and the cis and trans N-oxides of nicotine. It is preferred to maintain the total impurity content below about 1 wt% of the nicotine content of the nicotine device and most preferably as close to zero as possible.

The term, "nicotine device" as used herein, refers to a source of nicotine, typically an object such as, for example, a nicotine dosage form for use in smoking cessation which can be in the form of chewing gum, lozenge, tablet, suppository, transdermal delivery devices and the like and which include nicotine base.

The preferred pouching material is a laminate comprising a self-sealing nicotine barrier and a nicotine degradation agent barrier. The combination of self-sealing nicotine barrier and nicotine degradation agent barrier provides for simultaneous containment of the nicotine and protection of the nicotine from degradation.

The self-sealing nicotine barrier material is a nitrile rubber modified acrylonitrile-methyl acrylate copolymer (AN-MA/B). Preferably the self-sealing nicotine barrier material comprises the AN-MA/B material described above and the especially preferred nicotine barrier material comprises the 75AN-MA/B material described above. These materials are disclosed, for example, in U.S. Patent 3,426,102 which is incorporated herein by reference, and are commercially available as BAREX® resins in bulk and film form from numerous sources including Sohio Chemical Company, BP Chemicals International, Greenway Industries Corp., West Paterson, New Jersey, and Toyomenka (America) Inc., New York, New York, for example.

Various grades of 73-77 AN-MA/B are available, for example, BAREX 210, 214 and 218 of which BAREX 210 corresponds to the preferred 75AN-MA/B material.

The thickness of the AN-MA/B film used according to this invention will generally be from about 0.8 mil (20µ) or less to about 2.5 mil (65µ) or greater, and is preferably from about 1.0 mil (25µ) to about 2.0 mil (50µ). Thinner films can be used as long as the finished pouch is well-sealed and provides the requisite impermeability to the nicotine. Thicker films can be used as long as the sealing properties are not adversely affected, i.e., as long as the thicker films can be sealed properly with the heat-sealing equipment used. Films should not be so thick that the self-sealed portions (which are unprotected by a nicotine degradation agent barrier) provide a path for light, oxygen and/or water migration into the pouch.

While we have found AN-MA/B materials to be excellent nicotine barriers; they do not provide sufficient protection from nicotine degradation agents to function as a nicotine degradation agent barriers for the long-term containment of nicotine devices.

The transmission of oxygen by the preferred 75AN-MA/B material is reported to be as high as 0.8 cm³ mil/100 in^{2,} 24 hr., atm. (0.3 cm3·mm/m², 24 hr.) The water vapor transmission rate of this material is reported to be as high as 5.0 g mil/100 in², 24 hr. (2.0 g mm/m², 24 hr.) at 100°F (38°C) and 100% relative humidity. We have tested the preferred 75AN-MA/B material at 30°C, and at 10% and 90% relative humidity to determine the water uptake. After four hours the film at 10% RH had absorbed .32 wt.% water. After four hours the film at 90% RH had absorbed 2.4 wt.% water. These levels of oxygen transmission and water absorption render the preferred AN-MA/B material unsuitable, by itself, as a nicotine containment material.

AN-MA/B materials are available in untinted (light straw) and blue-tinted transparent film form and these materials do not provide sufficient light protection to prevent nicotine decomposition by light.

To provide an effective nicotine containment means according to our invention, the AN-MA/B nicotine barrier must be combined with a nicotine degradation agent barrier, which acts to inhibit the transmission of oxygen, water in liquid and vapor form, and light. The preferred nicotine degradation agent barrier is a metal film bonded to the 73-77 AN-MA/B nicotine barrier. Aluminum foil is preferred because of cost and availability.

It is also possible to vapor deposit aluminum or other metals directly onto AN-MA/B films and such materials could be used instead of metal foil provided the metal film so produced is sufficiently free of pin holes and irregularities to have the desired degradation agent barrier properties. If vapor deposited films are used, they offer the advantage of eliminating the adhesive or other bonding agent used to form the AN-MA/B aluminum foil laminate.

The thickness of the aluminum film is generally from less than about 0.35 mil (9µ) to about 1 mil (25µ) or greater. Films less than 0.35 mil (9µ) can be used when the film is of sufficient quality that irregularities and holes are minimized. Films thicker than 1 mil can be used, but tend to make the lamination process more difficult and the finished package somewhat stiffer.

In a preferred embodiment, the AN-MA/B nicotine barrier and an aluminum foil nicotine degradation agent barrier are bonded together to form a laminate. The laminate can be formed using a urethane adhesive such as Adcote™ 548 (Morton Chemical Co., Woodstock, Illinois) to bond the 73-77 AN-MA/B to aluminum foil.

It is also desirable to provide the laminate of this invention with an additional external protective lamina. This lamina is preferably formed from paper or a printable polymer such as polyethylene terephthalate (PET). Use of PET which is tear resistant offers the additional advantage of making the pouch more "child proof." This outer layer provides the finished pouch with a protective covering which prevents damage to the other layers is attractive, and is a good substrate for printing. Thickness is not critical as long as it does not interfere with the sealing process, and 35# kraft paper, 35# clay coat paper and 48 gauge (12µ) PET film are suitable. The protective layer, if present, can be adhered to the degradation agent lamina layer with any suitable adhesive such as polyethylene ethylene or an acrylic acetate (EAA) adhesive or to the nicotine barrier layer with an EAA adhesive, for example.

The nicotine containment pouches can be in any convenient form that permits the effective closure of the pouching materials and the formation of substantially complete nicotine and nicotine degradation agent barriers. The perimeter of the pouches can be, for example, oval, circular, triangular, irregularly shaped, square rectangular, and the like. For ease of manufacturing when the pouch is heat sealed, a square or rectangular shape is preferred.

The nicotine containment laminate can be self-sealed to form a nicotine containment pouch using any appropriate methods. 73-77 AN-MA/B film, for example, can be sealed using a variety of methods including heat (thermal bar) sealing, impulse sealing, radio frequency sealing, ultrasonic sealing, and the like.

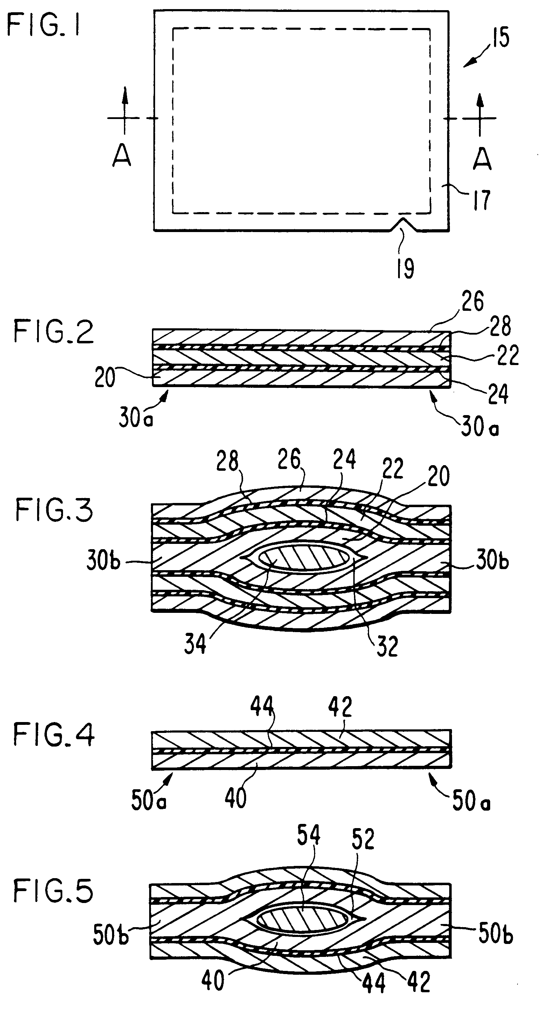

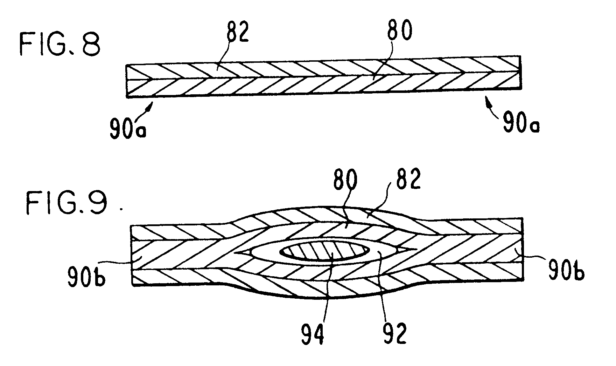

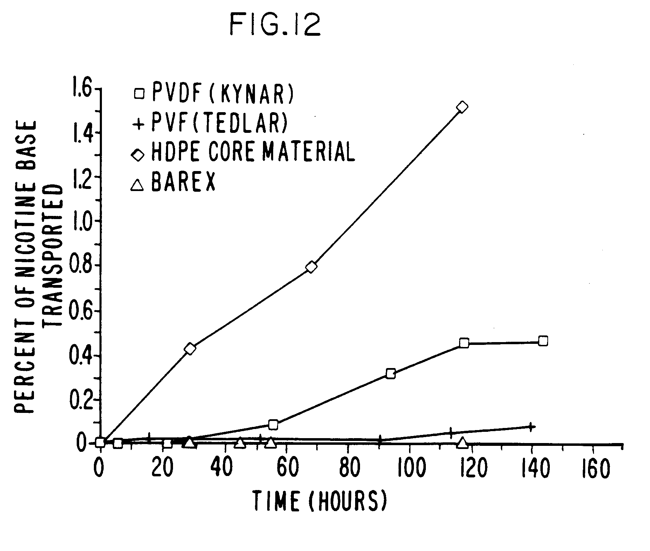

Referring now to the drawings, FIGURE 1 is a top view of a pouch 15 according to this invention. The edges 17 are self-sealed to form an enclosure which is substantially impermeable to nicotine and to nicotine degradation agents. A simple cut, or a notch 19 can be placed in the sealed edge area 17 to assist in opening the pouch.

FIGURE 2 is a cross-sectional view of a self-sealing laminate which is useful for producing pouches of this invention. The self-sealing nicotine barrier layer 20 is adhered to the nicotine degradation agent barrier layer 22 by an adhesive layer 24. protective coating layer 26 is adhered to the nicotine degradation agent barrier layer 22 by adhesive layer 28. The laminate can be self-sealed, for example, at edges 30a.

FIGURE 3 is a cross-sectional representation of the preferred embodiment of the pouch 15 of FIGURE 1, taken across line A-A. It is formed from the laminate of FIGURE 2. The self-sealing nicotine barrier layer 20 has been sealed at the edges 30b and forms an enclosure 32. Within the enclosure 32 is a nicotine device 34 such as a transdermal delivery device for the transport of nicotine base across the skin.

FIGURE 4 is a cross-sectional view of a an alternate self-sealing laminate which is useful for producing pouches of this invention. The self-sealing nicotine barrier layer 40 is adhered to the nicotine degradation agent barrier layer 42 by an adhesive layer 44. The laminate has self-sealable edges 50a.

FIGURE 5 is an alternate cross-sectional representation of the pouch 15 of FIGURE 1, taken across line A-A. It is formed from the laminate of FIGURE 4. The self-sealing nicotine barrier layer 40 has been sealed at the edges 50b and forms an enclosure 52. Within the enclosure 52 is a nicotine device 54.

FIGURE 8 is a cross-sectional view of an alternate self-sealing laminate. The self-sealing nicotine barrier layer 80 has been metallized and the nicotine degradation agent barrier layer 82 comprises a metallic film. The laminate has self-sealing edges 90a.

FIGURE 9 is a cross-sectional representation of the pouch 15 of FIGURE 1, taken across line A-A which has been formed from the laminate of FIGURE 8. The self-sealing nicotine barrier layer 80 has been sealed at the edges 90b and forms an enclosure 92. Within the enclosure 92 is a nicotine device 94.

The following examples are illustrative of the present invention. They are not to be construed as limitations of the scope of the invention. Variations and equivalents of these examples will be readily apparent to one skilled in the art in light of the present disclosure, and the claims herein. All percentages are weight percentages, and all temperatures are in degrees Celsius, unless otherwise noted.

### Example 1

5.0 ml nicotine base was heat-sealed within a pouch made of a 2 mil (50µ) film of the preferred 75AN-MA/B material described above. The outside dimensions of the pouch were approximately 3 cm by 6 cm. The pouch was placed in a bottle with 50 g. distilled water. The bottle was maintained at 51°C in a water bath.

The above procedure was repeated, substituting for the 75AN-MA/B films of the following materials: a 2 mil film of TEDLAR polyvinyl fluoride (PVF) available from duPont de Nemours, Wilmington, Delaware; a 3 mil film of KYNAR polyvinylidene fluoride (PVDF), Westlake Plastics Co., Lenni, Pennsylvania; a 2 mil film of high density polyethylene (HDPE) core material, stripped pouch stock, laminated with 1 mil aluminum foil and 35# Kraft paper (laminate provided by Richmond Technology, Redlands, California.) Nicotine migration through the pouches was determined, and is shown in Figure 12. All materials other than the AN-MA/B material showed excessive nicotine migration.

### Example 2

Films of various ACLAR chlorotrifluoroethylene polymers (CTFE), available from Allied Chemical Corp., Morristown, New Jersey, were exposed to nicotine in standard diffusion cells in an attempt to determine the permeability of such films to nicotine. 1.5 mil (38µ) and 3 mil (76µ) films of the copolymer ACLAR™ 22A, and 1 mil (25µ) film of the tripolymer ACLA® 33C were tested. The films all suffered failure due to nicotine induced stress cracks before the tests could be completed.

### Example 3

A 1.5 mil (38µ) thickness of the preferred 75AN-MA/B material (Greenway Industries Corp.) was laminated with 1 mil (25µ) aluminum foil using an Adcote 548 urethane adhesive with Catalyst F available from Morton Chemical Co., Woodstock, Illinois. The aluminum foil was then bonded to a 35# Kraft paper using 7# low density polyethylene (LDPE) to produce a laminate. The laminate was passed through a Circle Design (2-up) packager (Paxall Circle Design Machinery) having, at each sealing station, a metal heat bar at 425°F and an opposed rubber heat bar at 300°F. Sufficient pressure was applied to provide a good seal. A transdermal nicotine delivery device according to copending patent application serial number 06/906,730 was enclosed within the pouch. The nicotine device suffered no significant loss or degradation of its nicotine content when stored at 30°C for 18 months.

### Example 4

A 1.5 mil (38µ) thickness of the AN-MA/B material of Example 3 was laminated with 0.35 mil (9µ) aluminum foil using the adhesive of Example 3. The aluminum foil was then bonded to a 35# clay coat paper using 7# LDPE, to produce a laminate. The laminate was formed into a nicotine device containing pouch as in Example 3 above. The nicotine device suffered no significant loss or degradation of its nicotine content when stored at 30°C for 18 months.

### Example 5

A 2.5 mil (63µ) thickness of the AN-MA/B material of Example 3 was laminated with 0.35 mil (9µ) aluminum foil and 35# clay coat paper as described in Example 4. The laminate was formed into a nicotine device containing pouch as above. The nicotine device suffered no significant loss or degradation of its nicotine content when stored at 30°C for 18 months.

### Example 6

A laminate of 30µ of the AN-MA/B material of Example 3, 3µ urethane adhesive, 9µ aluminum, 20µ low density polyethylene adhesive and 58µ 35# clay coat paper was manufactured by Tomapoly Company, Ltd., Tokyo, Japan. When fabricated into nicotine containment pouches as described herein the pouches will be capable of stably containing nicotine for extended periods of time.

### Example 7

A pouch similar to that of Example 6 is formed by substituting 48 gauge (12µ) printable PET for the paper and 10 pound (17µ) EAA adhesive for the LDPE adhesive. The pouch is tear resistent and capable of stably containing nicotine for extended periods of time.

While the present invention has been described with respect to certain embodiments thereof, it should not be construed as limited thereto. It will be apparent to one skilled in the art that variations, modifications and substitutions can be made without departing from the scope of our invention, which is limited only by the following claims.

## Claims

1. A laminate pouch for the packaging of nicotine or a nicotine device (34; 54; 94), which pouch is made from a composite of nitrile rubber modified acrylonitrile-methyl acrylate copolymer and an opaque material impermeable to nicotine degrading agents, characterised in that said laminate comprises;
(i) first and second layers of said copolymer, each layer having a first and a second surface, and wherein portions (30a; 50a; 90a) of the second surface of the first layer juxtaposed the outer edges thereof are disposed in juxtaposition with corresponding portions of the second surface of the second layer in self-sealing contact, thereby to define self-sealed portions (30b; 50b; 90b) of an enclosure (32; 52; 92) for said nicotine or nicotine device (34; 54; 94), which enclosure lies between said first and second layers, and
(ii) top and bottom layers (22; 42; 82) of said opaque material, which top and bottom layers are juxtaposed in adhesive contact with the respective first surfaces of said copolymer first and second layers,
the arrangement being such that said self-sealed portions are unprotected by said opaque material.

2. A laminated pouch as claimed in claim 1, wherein the copolymer is formed from about 73-77 pbw acrylonitrile copolymerized with about 23-27 pbw methyl acrylate in the presence of about 8-10 pbw butadiene acrylonitrile copolymer containing about 70% by weight of polymer units derived from butadiene.

3. A laminated pouch as claimed in claim 1 wherein said copolymer is formed from about 75 pbw acrylonitrile and 25 pbw methyl acrylate copolymerized in the presence of about 10 parts by weight of the butadiene acrylonitrile copolymer containing about 70% by weight of polymer units derived from butadiene.

4. A laminated pouch as claimed in any one of claims 1 to 3 wherein said opaque material is a metal film.

5. A laminated pouch as claimed in claim 4 wherein said metal is aluminium.

6. A laminated pouch as claimed in any preceding claim and further comprising a protective layer (26) adhered to an external surface of said opaque material.

7. A laminated pouch as claimed in claim 6 wherein said protective layer is formed from a material selected from paper and polyethylene terephthalate.

8. A laminated pouch as claimed in any preceding claim wherein the first and second layers of copolymer are each from about 0.8 mil (20µ) to about 2.5 mil (64µ) thick.

9. A laminated pouch as claimed in any preceding claim wherein the opaque layer comprises aluminium foil having a thickness of from about 0.35 mil (9µ) to about 1 mil (25µ).

10. A method of forming a laminated pouch according to any preceding claim comprising:
(i) providing first and second layers of said copolymer, each layer having a first and a second surface;
(ii) adhering or vapour-depositing said opaque material on each layer's first surface;
(iii) placing the nicotine or nicotine device on either of said copolymer layers' second surfaces;
(iv) enclosing said nicotine or nicotine device by bringing said first and second layers' second surfaces into self-sealing contact at the edge portions of said layers,
the arrangement being such that said self-sealed edge portions are unprotected by opaque material.

11. A method of forming a laminated pouch as claimed in claim 10 wherein said first and second copolymer layers comprise a single sheet of copolymer, which sheet is folded to enclose said nicotine or nicotine device.

## Patentansprüche

1. Laminattasche zum Verpacken von Nikotin oder eines Nikotinproduktes, wobei die Tasche aus einem Verbundwerkstoff hergestellt ist, der aus mit Nitrilkautschuk modifiziertem Acrylnitrilmethylacrylat-Copolymer und einem für Nikotin abbauende Stoffe undurchlässigen opaken Material besteht, dadurch **gekennzeichnet,** daß das Laminat umfaßt:
i) eine erste und eine zweite Schicht des Copolymers, wobei jede Schicht eine erste und eine zweite Fläche hat und wobei Abschnitte (30a; 50a; 90a) der zweiten Fläche der ersten Schicht, die an die äußeren Ränder derselben angrenzen, an entsprechenden Abschnitten der zweiten Fläche der zweiten Schicht in selbstschließendem Kontakt anliegen, um dadurch selbstschließende Abschnitte (30b; 50b; 90b) einer Hülle (32; 52, 92) für das Nikotin oder Nikotinprodukt (34; 54; 94) zu bilden: die zwischen der ersten und der zweiten Schicht liegt und
ii) eine Grund- und eine Deckschicht (22; 42; 82) aus dem opaken Material, die mit klebendem Kontakt an der jeweiligen ersten Fläche der ersten und zweiten Schicht anliegen,
wobei die Anordnung so getroffen ist, daß die selbstschließenden Abschnitte von dem opaken Material nicht geschützt sind.

2. Laminierte Tasche nach Anspruch 1, wobei das Copolymer aus circa 73 bis 77 Gew.-% Akrylnitril, copolymisiert mit ca. 23 bis 27 Gewichtsteilen Metylakrylat im Beisein von circa 8 bis 10 Gewichtsteilen Butadienakrylnitril-Copolymer hergestellt wird, das circa 70 Gew.-% von Butadien abgeleiteten Polymereinheiten enthält.

3. Laminierte Tasche nach Anspruch 1, wobei das Copolymer aus circa 75 Gewichtsteilen Akrylnitril und 25 Gewichtsteilen Metylakrylat, copolymisiert im Beisein von circa 10 Gewichtsteilen Butadienakrylnitril-Copolymer hergestellt wird, das circa 70 Gew.-% von aus Butadien abgeleiteten Polymereinheiten enthält.

4. Laminierte Tasche nach einem der Ansprüche 1 bis 3, wobei das opake Material ein Metallfilm ist.

5. Laminierte Tasche nach Anspruch 4, wobei das Metall Aluminium ist.

6. Laminierte Tasche nach einem der vorangegangenen Ansprüche, **gekennzeichnet** durch eine Schutzschicht (26), die an der Außenfläche des opaken Materials haftet.

7. Laminierte Tasche nach Anspruch 6, wobei die Schutzschicht aus einem Material hergestellt wird, das aus einer Papier und Polyäthylenterephtalat enthaltenden Materialgruppe ausgewählt wurde.

8. Laminierte Tasche nach einem der vorangegangenen Ansprüche, wobei die erste und die zweite Copolymerschicht jeweils eine Dicke von 0,8 mil (20µ) bis circa 2,5 mil (64µ) haben.

9. Laminierte Tasche nach einem der vorangegangenen Ansprüche, wobei die opake Schicht eine Aluminiumfolie mit einer Dicke von circa 0,35 mil (9µ) bis circa 1 mil (25µ) hat.

10. Verfahren zur Herstellung einer laminierten Tasche nach einem der vorangegangenen Ansprüche, umfassend folgende Schritte:
(i) Vorsehen einer ersten und einer zweiten Copolymerschicht, von denen jede eine erste und eine zweite Fläche hat;
(ii) Anbringen oder Aufdampfen eines opaken Materials auf jeder der ersten Schichtflächen;
(iii) Anordnen des Nikotins oder Nikotinproduktes auf einer der zweiten Flächen der Copolymerschichten;
(iv) Einschließen des Nikotins oder Nikotinproduktes, indem die zweiten Flächen der ersten und zweiten Schicht im Randbereich dieser Schichten in selbstschließenden Kontakt gebracht werden,
wobei die Anordnung so getroffen ist daß die selbstschließenden Randabschnitte durch das opake Material nicht geschützt sind.

11. Verfahren zum Herstellen einer laminierten Tasche nach Anspruch 10, wobei die erste und die zweite Copolymerschicht aus einer einzigen Copolymerbahn bestehen, die gefaltet wird, um das Nikotin oder Nikotinprodukt einzuschließen.

## Revendications

1. Sachet stratifié pour emballer de la nicotine ou un dispositif à nicotine (34 ; 54 ; 94), ce sachet étant fait en un composite de copolymère acrylonitrile modifié par un caoutchouc de nitrile/acrylate de méthyle et d'un matériau opaque imperméable aux agents dégradant la nicotine, caractérisé en ce que ledit stratifié comprend :
(i) des première et deuxième couches dudit copolymère, chaque couche ayant une première et une deuxième surface, et où des parties (30a ; 50a ; 90a) de la deuxième surface de la première couche juxtaposées sur ses bords extérieurs sont disposées en juxtaposition avec des parties correspondantes de la deuxième surface de la deuxième couche en contact d'auto-scellement, de façon à définir des parties auto-scellées (30b ; 50b ; 90b) d'une enceinte (32 ; 52 ; 92) pour ladite nicotine ou ledit dispositif à nicotine (34 ; 54 ; 94), ladite enceinte 0se trouvant entre lesdites première et deuxième couches, et
(ii) des couches supérieure et inférieure (22 ; 42 ; 82) dudit matériau opaque, ces couches supérieure et inférieure étant juxtaposées en contact d'adhérence avec les premières surfaces respectives desdites première et deuxième couches de copolymère,
l'agencement étant tel que lesdites parties auto-scellées ne sont pas protégées par ledit matériau opaque.

2. Sachet stratifié selon la revendication 1, dans lequel le copolymère est formé d'environ 73 à 77 parties en poids d'acrylonitrile copolymérisé avec environ 23 à 27 parties en poids d'acrylate de méthyle en présence d'environ 8 à 10 parties en poids de copolymère butadiène/acrylonitrile contenant environ 70 % en poids de motifs polymères dérivant du butadiène.

3. Sachet stratifiée selon la revendication 1, dans lequel ledit copolymère est formé d'environ 75 parties en poids d'acrylonitrile et de 25 parties en poids d'acrylate de méthyle copolymérisés en présence d'environ 10 parties en poids du copolymère butadiène/acrylonitrile contenant environ 70 % en poids de motifs polymères dérivant du butadiène.

4. Sachet stratifié selon l'une quelconque des revendications 1 à 3, dans lequel ledit matériau opaque est un film métallique.

5. Sachet stratifié selon la revendication 4, dans lequel ledit métal est de l'aluminium.

6. Sachet stratifié selon l'une quelconque des revendications précédentes, et comprenant en outre une couche protectrice (26) adhérant à une surface externe dudit matériau opaque.

7. Sachet stratifié selon la revendication 6, dans lequel ladite couche protectrice est formée en un matériau choisi parmi le papier et le poly(éthylènetéréphtalate).

8. Sachet stratifié selon l'une quelconque des revendications précédentes, dans lequel les première et deuxième couches de copolymères ont chacune une épaisseur d'environ 20 µm (0,8 mil) à environ 64 µm (2,5 mils).

9. Sachet stratifié selon l'une quelconque des revendications précédentes, dans lequel la couche opaque comprend une feuille d'aluminium ayant une épaisseur d'environ 9 µm (0,35 mil) à environ 25 µm (1 mil).

10. Procédé pour former un sachet stratifié selon l'une quelconque des revendications précédentes, qui consiste à :
(i) disposer d'une première et d'une deuxième couches dudit copolymère, chaque couche ayant une première et une deuxième surfaces ;
(ii) faire adhérer ou déposer en phase vapeur ledit matériau opaque sur chaque première surface de couche ;
(iii) placer la nicotine ou le dispositif à nicotine sur l'une quelconque desdites deuxièmes surfaces des couches de copolymère ;
(iv) enfermer ladite nicotine ou ledit dispositif à nicotine par mise en contact d'auto-scellement desdites deuxièmes surfaces des première et deuxième couches au niveau des parties de bord desdites couches,
l'agencement étant tel que lesdites parties de bord auto-scellées ne sont pas protégées par le matériau opaque.

11. Procédé pour former un sachet stratifié selon la revendication 10, dans lequel lesdites première et deuxième couches de copolymère comprennent une feuille unique de copolymère, cette feuille étant pliée de façon à renfermer ladite nicotine ou ledit dispositif à nicotine.
